# EUROPÄISCHE PATENTSCHRIFT

(11) **EP 1 926 704 B1**
(45) Veröffentlichungstag und Bekanntmachung des Hinweises auf die Patenterteilung: **01.04.2009**
(21) Anmeldenummer: 06793474.5
(22) Anmeldetag: 13.09.2006
(51) Int. Cl.: C07C 253/30, C07C 255/24

(54) **VERFAHREN ZUR HERSTELLUNG VON BETA-AMINOPROPIONSÄUREDERIVATEN**
METHOD FOR THE PRODUCTION OF BETA-AMINOPROPIONIC ACID DERIVATIVES
PROCEDE POUR PRODUIRE DES DERIVES D'ACIDE BETA-AMINOPROPIONE

(30) Priorität: 15.09.2005 DE 102005044090; 21.02.2006 EP 06110212
(43) Veröffentlichungstag der Anmeldung: 04.06.2008
(73) Patentinhaber: BASF SE, 67056 Ludwigshafen (DE)
(72) Erfinder: EVERS, Holger, 67063 Ludwigshafen (DE); MELDER, Johann-Peter, 67459 Böhl-Iggelheim (DE); PREISS, Thomas, 67256 Weisenheim (DE); MEISSNER, Harald, 67454 Hassloch (DE)
(74) Vertreter: Isenbruck, Günter
(86) Internationale Anmeldenummer: PCT/EP2006/066311
(87) Internationale Veröffentlichungsnummer: WO 2007/031534

(56) Entgegenhaltungen:
- EP-A1- 0 630 886
- GB-A- 613 807

## Beschreibung

Die Erfindung betrifft ein Verfahren zur Herstellung von β-Aminopropionsäurederivaten durch Umsetzung eines primären oder sekundären Amins mit einem Acrylsäurederivat.

Die Anlagerung von Aminen an Acrylsäurederivate wie Acrylnitril stellt einen klassischen Fall der Addition von Nukleophilen an vinyloge Carbonylverbindungen dar. Das Gleichgewicht zwischen Amin und Acrylsäurederivat einerseits und dem β-Aminopropionsäurederivat andererseits liegt weit auf der Produktseite. Da es sich um eine Gleichgewichtsreaktion handelt, ist bei einem Eduktverhältnis von 1 : 1 kein Vollumsatz der Edukte zu erzielen.

Acrylsäurederivate sind toxisch, cancerogen und neigen zu stark exothermer Polymerisation. Rohprodukte mit einem Acrylnitril-Restgehalt von > 1000 ppm sind als toxisch einzustufen. Handhabung, Transport und Aufarbeitung derartiger Gemische sind nur unter besonderen Vorkehrungen und in geeigneten Anlagen zulässig. Um erhöhte Konzentrationen von nicht umgesetzten Acrylsäurederivaten in den Rohprodukten zu vermeiden, wird die Anlagerung von Amin an das Acrylsäurederivat in der Regel mit einem Amin-Überschuss durchgeführt. Ein Vollumsatz der unter Umständen wertvollen Aminkomponente ist dann nicht möglich. Aus dem Rohproduktgemisch schlecht abtrennbare, wertvolle Amine gehen dadurch verloren.

DD-A 58 306 offenbart ein Verfahren zur Herstellung von 3-Dimethylaminopropionsäurenitril, bei dem 3-Dimethylaminopropionsäurenitril vorgelegt, bei Temperaturen von 10 bis 20 °C mit Dimethylamin gesättigt und anschließend bei etwa 20 °C die dem Dimethylamin entsprechende Menge Acrylnitril unter Kühlung zugefügt wird. Nach Zugabe des Acrylnitrils wird wieder wechselweise Dimethylamin und Acrylnitril zugegeben. Von Zeit zu Zeit wird das Reaktionsprodukt aus dem Reaktionsgefäß abgetrennt und der Destillation zugeführt.

DD-A 222 011 offenbart ein Verfahren zur kontinuierlichen Herstellung von 3-N,N-Dimethylaminopropionitril aus Dimethylamin und Acrylnitril bei Temperaturen zwischen 25 und 100 °C, bei dem Dimethylamin zu Acrylnitril in einem Molverhältnis von vorzugsweise 1 : 1,05 bis 1,05 : 1 eingesetzt wird, wobei die Reaktionskomponenten wässrig oder wasserfrei eingesetzt werden können. Besonders hohe Raum-Zeit-Ausbeuten werden laut Beschreibung in Gegenwart von Wasser erreicht.

Es sind verschiedene Techniken bekannt, um Acrylnitril aus Acrylnitril enthaltenden Rohprodukten zu entfernen.

DD-A 144 765 offenbart ein Verfahren zur Entfernung des noch enthaltenen Acrylnitrils aus Roh-Acetonitril nach der Azeotropdestillation, bei dem dem Roh-Acetonitril ein aliphatisches Amin, vorzugsweise Ethanolamin, in Mengen von 0,1 bis 5,0 %, bezogen auf das Roh-Acetonitril zugesetzt wird. Nach den Angaben in der Beschreibung reagiert speziell Ethanolamin bei 50 bis 90 °C mit Acrylnitril unter Bildung von Verbindungen mit hohem Siedepunkt, welche sich destillativ abtrennen lassen, worauf ein reines Acetonitril gewonnen werden kann.

DE-A 33 34 328 offenbart ein Verfahren zur Vernichtung von gebundener Blausäure und Acrylnitril in rohem Acetonitril, bei dem das rohe Acetonitril, gegebenenfalls nach destillativer Abtrennung und Gewinnung von freier Blausäure, mit einer Base bei einem pH-Wert von 8,5 bis 11, einer Temperatur von 200 bis 250 °C und einer Verweilzeit von 3 bis 20 Minuten umgesetzt wird. Als Basen werden Alkali- oder Erdalkalihydroxide, insbesondere NaOH genannt, die in Form ihrer wässrigen Lösungen zugesetzt werden.

Bekannt ist ferner, Acrylnitril aus Polymeremulsionen durch Umsetzung mit Oximen (US 4,365,027), Hydroxylamin (EP-A 0 204 953) und heterocyclischen Aminen (US 4,399,240) zu entfernen. GB 613, 807 offenbart die destillative Abtrennung der nicht umgesetzten Komponenten.

Die Abtrennung von überschüssigem Acrylnitril durch Destillation im technischen Vakuum (ca. 20 bis 30 mbar) erfordert hohe Temperaturen und lange Verweilzeiten. Dabei werden farbgebende Nebenkomponenten gebildet, deren Abtrennung aus hochsiedenden Wertprodukten im Allgemeinen nicht möglich ist.

Soll beispielsweise zur Verringerung des Rest-Acrylnitrilgehalts Acrylnitril aus dem Rohprodukt der Addition von Amin an Acrylnitril im Vakuum abgetrennt werden, müssen die Temperaturen unter 60 °C bleiben, da sich andernfalls durch Bildung farbgebender Nebenprodukte die Farbzahl des Produktes deutlich verschlechtert. Um unter diesen Bedingungen Rest-Acrylnitrilgehalte von < 1000 ppm zu erzielen, sind sehr lange Verweilzeiten im Vakuum erforderlich.

Aufgabe der Erfindung ist es, ein Verfahren zur Herstellung von β-Aminopropionsäurederivaten durch Umsetzung eines primären oder sekundären Amins als Wertamin mit einem Acrylsäurederivat bereitzustellen, bei dem ein Rohprodukt erhalten wird, welches durch sehr geringe Gehalte an nicht umgesetztem Acrylsäurederivat gekennzeichnet ist. Insbesondere soll ein derartiges Verfahren bereitgestellt werden, bei dem das Wertamin weitgehend, vorzugsweise im Wesentlichen vollständig umgesetzt wird und dennoch ein Rohprodukt erhalten wird, welches durch sehr geringe Gehalte an nicht umgesetztem Acrylsäurederivat gekennzeichnet ist.

Gelöst wird die Aufgabe durch ein Verfahren zur Herstellung von β-Aminopropionsäurederivaten durch Umsetzung eines primären oder sekundären Amins mit einem Acrylsäurederivat, wobei
(i) ein erstes primäres oder sekundäres Amin als Wertamin bereitgestellt und mit dem Acrylsäurederivat umgesetzt wird, wobei ein Reaktionsgemisch enthaltend ein erstes β-Aminopropionsäurederivat als Wertprodukt und daneben nicht umgesetztes Acrylsäurederivat erhalten wird,
(ii) das in dem Reaktionsgemisch enthaltene nicht umgesetzte Acrylsäurederivat mit einem zweiten sekundären Amin als Abfangamin praktisch vollständig zu einem zweiten β-Aminopropionsäurederivat umgesetzt wird, wobei ein Reaktionsgemisch enthaltend das erste β-Aminopropionsäurederivat als Wertprodukt, das zweite β-Aminopropionsäurederivat und nicht umgesetztes sekundäres Amin erhalten wird.

In einer bevorzugten Ausführungsform des erfindungsgemäßen Verfahrens wird das Acrylsäurederivat bezüglich der primären beziehungsweise sekundären Aminogruppen des Wertamins in stöchiometrischem Überschuss eingesetzt, wobei im Wesentlichen das gesamte Wertamin reagiert.

Diese Variante zeichnet sich dadurch aus, dass das primäre oder sekundäre Wertamin praktisch vollständig zum Wertprodukt umgesetzt wird. Da das Acrylsäurederivat in stöchiometrischem Überschuss eingesetzt wird, sind ferner bereits vergleichsweise milde Reaktionsbedingungen ausreichend, um dennoch in akzeptablen Reaktionszeiten praktisch einen Vollumsatz des Wertamins zu erzielen. Durch milde Reaktionsbedingungen wird die Bildung farbgebender Nebenprodukte unterdrückt.

In einer weiteren Ausführungsform wird das Acrylsäurederivat bezüglich der primären beziehungsweise sekundären Aminogruppen des Wertamins im Wesentlichen äquimolar eingesetzt. Auch hierbei wird noch ein weitgehender Umsatz des Wertamins erzielt. In einer weiteren Ausführungsform wird das Acrylsäurederivat bezüglich der primären beziehungsweise sekundären Aminogruppen unterstöchiometrisch eingesetzt.

In jedem Fall werden durch die Reaktion mit dem Abfangamin in Schritt (ii) des erfindungsgemäßen Verfahrens sehr niedrige Restkonzentrationen des Acrylsäurederivats in dem Endprodukt erreicht, ohne dass ein hoher Überschuss des Wertamins eingesetzt werden muss. Eine Abtrennung des Acrylsäurederivats durch Destillation, welche hohe Temperaturen und lange Verweilzeiten erfordert und zur Bildung von farbgebenden Nebenkomponenten führt, entfällt somit.

Beispiele für Wertamine, welche nach dem erfindungsgemäßen Verfahren umgesetzt werden können, sind 3,3'-Dimethyl-4,4'-diaminodicyclohexylmethan (CAS-Nr. 6864-37-5), 4,4'-Diaminodicyclohexylmethan (CAS-Nr. 1761-71-3), 4,4'-Diaminodiphenylmethan (CAS-Nr. 101-77-9), Hexamethylendiamin (CAS-Nr. 124-09-4), 2-Methylpentamethylendiamin (CAS-Nr. 15520-10-2); Polyetheramin D 230 (CAS-Nr. 9046-10-0) und 1,13-Diamino-4,7,10-trioxatridecan (CAS-Nr. 4246-51-9).

Wird das Acrylsäurederivat in stöchiometrischem Überschuss eingesetzt, so wird es im Allgemeinen in einem Überschuss von 0,1 bis 10 Mol-%, vorzugsweise 0,2 bis 2 Mol-%, bezogen auf die reagierenden primären beziehungsweise sekundären Aminogruppen des Wertamins, eingesetzt, d.h. pro Mol primärer beziehungsweise sekundärer Aminogruppen werden 1,001 bis 1,10 Mol, vorzugsweise 1,002 bis 1,02 Mol des Acrylsäurederivats eingesetzt. Die Menge an eingesetztem Acrylsäurederivat kann selbstverständlich auch darüber liegen.

Das Acrylsäurederivat kann jedoch auch in stöchiometrischem Unterschuss eingesetzt werden, beispielsweise von bis zu 10 Mol-% (d. h. pro Mol reagierender Aminogruppen des Wertamins werden nur 0,9 Mol des Acrylsäurederivats eingesetzt).

Das Acrylsäurederivat kann Acrylnitril oder ein Acrylsäureester sein. Übliche Acrylsäureester, welche nach dem erfindungsgemäßen Verfahren umgesetzt werden können, sind die C₁-C₆-Alkylacrylate, vorzugsweise Methylacrylat und Ethylacrylat. Bevorzugtes Acrylsäurederivat ist Acrylnitril. Geeignete Abfangamine (Skavangeramine), welche in Schritt (ii) mit dem in dem Reaktionsgemisch enthaltenen nicht umgesetzten Acrylsäurederivat umgesetzt werden, sind niedrigsiedende oder hochsiedende sekundäre Amine. Bevorzugte niedrigsiedende sekundäre Amine weisen einen Siedepunkt von bis zu 100 °C auf. Solche niedrigsiedenden Amine können aus dem Reaktionsgemisch der Stufe (ii), welches das Wertprodukt, das weitere β-Aminopropionsäurederivat und nicht umgesetztes sekundäres Amin enthält, ohne weiteres destillativ abgetrennt werden. Geeignete niedrigsiedende sekundäre Amine sind beispielsweise die Di-(C₁-C₄-alkyl)amine. Besonders bevorzugtes, als Abfangamin eingesetztes sekundäres Amin ist Dimethylamin. Weitere Beispiele sind Diethylamin, Diisopropylamin und Dibutylamin. Das Abfangamin kann als Flüssigkeit, wässrige Lösung oder auch gasförmig zugegeben werden.

Überschüssiges Abfangamin kann aber auch in dem Reaktionsgemisch verbleiben. In diesem Fall wird das Abfangamin eher ein hochsiedendes sekundäres Amin sein. Beispiele sind N-Ethyltoluidin und Dicyclohexylamin.

Das durch Reaktion von nicht umgesetztem Acrylsäurederivat und Abfangamin gebildete zweite β-Aminopropionsäurederivat kann ebenfalls entweder aus dem Reaktionsgemisch destillativ abgetrennt werden oder in dem Reaktionsgemisch verbleiben. Für viele Anwendungen des als Wertprodukt erhaltenen β-Aminopropionsäurederivats stört die Anwesenheit von geringen Mengen des in Schritt (ii) gebildeten zweiten β-Aminopropionsäurederivats nicht, so dass dies ohne weiteres in dem Produktgemisch verbleiben kann. Beispiele solcher Anwendungen sind die Verwendung des Wertproduktes als Härter für Polyurethane oder Epoxidharze.

Somit umfasst also das erfindungsgemäße Verfahren die Varianten, wonach aus dem in Schritt (ii) erhaltenen Reaktionsgemisch
(iii) das nicht umgesetzte sekundäre Amin destillativ abgetrennt wird
   und/oder
(iv) das zweite β-Aminopropionsäurederivat destillativ abgetrennt wird,
wobei keiner, nur einer oder beide der Schritte (iii) und (iv) durchgeführt werden können.

Wie bereits erwähnt, kann die Umsetzung des primären beziehungsweise sekundären Wertamins mit dem Acrylsäurederivat zum β-Aminopropionsäurederivat unter milden Bedingungen durchgeführt werden. So kann Schritt (i) in Gegenwart von Wasser als Katalysator bei einer Reaktionstemperatur von 20 bis 80 °C, vorzugsweise 40 bis 60 °C durchgeführt werden, wobei wegen des Acrylsäurederivat-Überschusses akzeptable Reaktionsgeschwindigkeiten und Vollumsatz des Wertamins auch unter diesen milden Reaktionsbedingungen erzielt werden. Die Reaktion von überschüssigem Acrylsäurederivat und Abfangamin in Schritt (ii) des erfindungsgemäßen Verfahrens kann unter den gleichen milden Bedingungen wie die Hauptreaktion in Schritt (i) erfolgen, wobei kein weiterer Zusatz von Wasser erforderlich ist. Durch Wahl eines geeigneten Überschusses an Abfangamin über nicht umgesetztes Acrylsäurederivat kann sichergestellt werden, dass die Abfangreaktion in Schritt (ii) praktisch vollständig ist. Dabei kann die Restkonzentration des Acrylsäurederivats auf Konzentrationen unterhalb der Nachweisgrenze (ca. 50 ppm) reduziert werden. Im Allgemeinen wird, bezogen auf nicht umgesetztes Acrylsäurederivat, das 1,5 bis 10-fache, bevorzugt das 1,5 bis 2-fache an Abfangamin (Molverhältnis) eingesetzt.

In Gegenwart des nicht umgesetzten Überschusses des sekundären Abfangamins steigt auch die Lagerfähigkeit des Rohproduktes, da die durch Retro-Michael-Reaktion freigesetzten Acrylsäurederivate in situ abgefangen werden. Die Gefahr farbgebender oder stark exothermer Polymerisationsreaktionen wird durch die Unterdrückung freier Acrylsäurederivate in dem Rohprodukt stark vermindert.

Eine Aufarbeitung des Rohproduktes zur Isolierung des Wertproduktes durch destillative Abtrennung von. Leichtsiedern (Wasser, nicht umgesetztes Abfangamin) beziehungsweise des weiteren β-Aminopropionsäurederivats ist auch bei höheren Temperaturen möglich, da kein freies Acrylsäurederivat mehr in dem Rohproduktgemisch vorhanden ist.

In einer besonders bevorzugten Ausführungsform wird das erfindungsgemäße Verfahren eingesetzt, um Isophorondiamin mit zwei Äquivalenten Acrylnitril zu N,N-Biscyanoethylisophorondiamin umzusetzen. Das Verfahren kann beispielsweise wie folgt durchgeführt werden:

Isophorondiamin (IPDA) und ca. 10 Gew.-% Wasser, bezogen auf IPDA, werden in einem Rührkessel vorgelegt und 2,002 bis 2,10, beispielsweise 2,02 Äquivalente Acrylnitril bei 20 bis 50 °C, beispielsweise 30 bis 40 °C zugetropft. Anschließend wird noch einige Stunden bei 50 bis 70 °C, beispielsweise ca. 60 °C weiter gerührt. Anschließend werden 1,5 bis 2 Äquivalente, bezogen auf nicht umgesetztes Acrylnitril, Dimethylamin (DMA) zugegeben. DMA kann als wässrige Lösung zugegeben oder als Gas eingeleitet werden. Anschließend wird noch einige Stunden weiter gerührt, bis Acrylnitril praktisch vollständig umgesetzt ist. Sodann werden Wasser und überschüssiges DMA im Vakuum bei 20 bis 30 mbar und 50 bis 60 °C abgetrennt. Es wird eine klare Flüssigkeit erhalten, die < 100 ppm IPDA und < 50 ppm Acrylnitril enthält.

Die Erfindung wird durch die nachstehenden Beispiele näher erläutert.

### Beispiele

### Beispiel 1

In einem geeigneten Gefäß werden 1 Mol IPDA und 10 Gew.-% Wasser, bezogen auf IPDA, vorgelegt. Unter Rühren und Temperaturkontrolle werden 2,2 Mol Acrylnitril bei < 60 °C zudosiert. Nach Vollumsatz des IPDA (> 98% Biscyanoethyl-IPDA) wird der Acrylnitril-Restgehalt gaschromatographisch bestimmt. Anschließend werden bei einer Temperatur < 60 °C pro Mol Rest-Acrylnitril 2 Mol Dimethylamin zudosiert und gerührt, bis das Acrylnitril vollständig umgesetzt ist (Rest-Acrylnitrilgehalt unterhalb der Nachweisgrenze). In einer geeigneten Apparatur, beispielsweise einem Rotationsverdampfer, werden unter Vakuum bei ca. 30 mbar die Leichtsieder (Wasser, Dimethylamin und β-Diaminopropionitril) entfernt. Aufgrund der milden Reaktions- und Aufarbeitungsbedingungen wird ein farbloses Produkt (Farbzahl < 10 APHA) erhalten.

### Vergleichsbeispiel 1

Es werden, analog Beispiel 1, 1,0 Mol IPDA mit 2,2 Mol Acrylnitril unter Wasserkatalyse umgesetzt. Nach Beendigung der Reaktion werden die Leichtsieder (Wasser und Acrylnitril) im Vakuum bei ca. 30 mbar abgezogen. Bei Erreichen eines Acrylnitril-Restgehalts von < 1000 ppm liegt ein gelbliches Produkt (Farbzahl 160 APHA) vor.

### Vergleichsbeispiel 2

Analog Beispiel 1 werden 1,0 Mol IPDA mit nur 2,0 Mol Acrylnitril unter Wasserkatalyse umgesetzt. Nach Beendigung der Reaktion beträgt der Acrylnitril-Restgehalt noch > 1000 ppm. Es gelingt unter den milden Reaktionsbedingungen nicht, den Acrylnitril-Restgehalt weiter abzusenken. Nach Abtrennung von nicht umgesetztem Acrylnitril im Vakuum bei ca. 30 mbar wird ein gelbliches Produkt (Farbzahl 176 APHA) erhalten.

### Beispiel 2

Es wird analog Beispiel 1 verfahren, jedoch werden nur 2,0 Mol Acrylnitril mit 1 Mol IPDA umgesetzt. Nach Vollumsatz des IPDA (> 98% Biscyanoethyl-IPDA) wird der Acrylnitrilgehalt ermittelt und pro Mol Rest-Acrylnitril 2 Mol Dimethylamin zugesetzt. Im Übrigen wird wie in Beispiel 1 aufgearbeitet. Es wird ein farbloses Produkt (Farbzahl < 10 APHA) erhalten.

### Beispiel 3

Es wird wie in den Beispielen 1 und 2 verfahren, jedoch werden nur 1,9 Mol Acrylnitril mit 1 Mol IPDA umgesetzt. Nach Umsatz des IPDA zu Monocyanoethyl- und Biscyanoethyl-IPDA wird der Rest-Acrylnitrilgehalt ermittelt und pro Mol Rest-Acrylnitril 2 Mol Dimethylamin zugesetzt. Im Übrigen wird wie in den Beispielen 1 und 2 aufgearbeitet. Es wird ein farbloses Produkt (Farbzahl < 10 APHA) erhalten.

## Patentansprüche

1. Verfahren zur Herstellung von β-Aminopropionsäurederivaten durch Umsetzung eines primären oder sekundären Amins mit einem Acrylsäurederivat, **dadurch gekennzeichnet, dass**
(i) ein erstes primäres oder sekundäres Amin als Wertamin bereitgestellt und mit dem Acrylsäurederivat umgesetzt wird, wobei ein Reaktionsgemisch enthaltend ein erstes β-Aminopropionsäurederivat als Wertprodukt und daneben nicht umgesetztes Acrylsäurederivat erhalten wird,
(ii) das in dem Reaktionsgemisch enthaltene nicht umgesetzte Acrylsäurederivat mit einem zweiten sekundären Amin als Abfangamin praktisch vollständig zu einem zweiten β-Aminopropionsäurederivat umgesetzt wird, wobei ein Reaktionsgemisch enthaltend das erste β-Aminopropionsäurederivat als Wertprodukt, das zweite β-Aminopropionsäurederivat und nicht umgesetztes sekundäres Amin erhalten wird.

2. Verfahren nach Anspruch 1, **dadurch gekennzeichnet, dass** das Acrylsäurederivat bezüglich der primären beziehungsweise sekundären Aminogruppen des Wertamins in stöchiometrischem Überschuss eingesetzt wird.

3. Verfahren nach Anspruch 1, **dadurch gekennzeichnet, dass** das Acrylsäurederivat bezüglich der primären beziehungsweise sekundären Aminogruppen des Wertamins im Wesentlichen äquimolar oder in stöchiometrischem Unterschuss eingesetzt wird.

4. Verfahren nach einem der Ansprüche 1 bis 3, **dadurch gekennzeichnet, dass** aus dem in Schritt (ii) erhaltenen Reaktionsgemisch
(iii) das nicht umgesetzte sekundäre Amin destillativ abgetrennt wird,
und/oder
(iv) das zweite β-Aminopropionsäurederivat destillativ abgetrennt wird.

5. Verfahren nach einem der Ansprüche 1 bis 4, **dadurch gekennzeichnet, dass** das Acrylsäurederivat Acrylnitril oder ein Acrylsäureester ist.

6. Verfahren nach Anspruch 4 oder 5, **dadurch gekennzeichnet, dass** das in Schritt (ii) eingesetzte sekundäre Amin ein leichtsiedendes Amin mit einem Siedepunkt bis zu 100 °C ist und Schritt (iii) durchgeführt wird.

7. Verfahren nach einem der Ansprüche 1 bis 6, **dadurch gekennzeichnet, dass** das in Schritt (ii) eingesetzte sekundäre Amin ein Di-(C₁-C₄-alkyl)amin ist.

8. Verfahren nach einem der Ansprüche 1 bis 7, **dadurch gekennzeichnet, dass** das in Schritt (ii) eingesetzte sekundäre Amin Dimethylamin ist.

9. Verfahren nach einem der Ansprüche 1 bis 8, **dadurch gekennzeichnet, dass** das Wertamin ausgewählt ist aus der Gruppe bestehend aus 3,3'-Dimethyl-4,4'-diaminodicyclohexylmethan (CAS-Nr. 6864-37-5), 4,4'-Diaminodicyclohexylmethan. (CAS-Nr. 1761-71-3), 4,4'-Diaminodiphenylmethan (CAS-Nr. 101-77-9), Hexamethylendiamin (CAS-Nr. 124-09-4), 2-Methylpentamethylendiamin (CAS-Nr. 15520-10-2), Polyetheramin D 230 (CAS-Nr. 9046-10-0) und 1,13-Diamino-4,7,10-trioxatridecan (CAS-Nr. 4246-51-9).

10. Verfahren nach einem der Ansprüche 1 bis 9, **dadurch gekennzeichnet, dass** Schritt (i) in Gegenwart von Wasser als Katalysator bei einer Temperatur von 20 bis 80 °C durchgeführt wird.

## Claims

1. A process for preparing β-aminopropionic acid derivatives by reacting a primary or secondary amine with an acrylic acid derivative, which comprises
(i) providing a first primary or secondary amine as an amine of value and reacting it with the acrylic acid derivative, to obtain a reaction mixture comprising a first β-aminopropionic acid derivative as a product of value and additionally unconverted acrylic acid derivative,
(ii) reacting the unconverted acrylic acid derivative present in the reaction mixture with a second secondary amine as a scavenger amine virtually fully to give a second β-aminopropionic acid derivative to obtain a reaction mixture comprising the first β-aminopropionic acid derivative as a product of value, the second β-aminopropionic acid derivative and unconverted secondary amine.

2. The process according to claim 1, wherein the acrylic acid derivative is used in stoichiometric excess with regard to the primary or secondary amino groups of the amine of value.

3. The process according to claim 1, wherein the acrylic acid derivative is used in an essentially equimolar amount or in a stochiometric deficiency with regard to the primary or secondary amino groups of the amine of value.

4. The process according to any of claims 1 to 3, wherein, from the reaction mixture obtained in step (ii),
(iii) the unconverted secondary amine is removed by distillation,
and/or
(iv) the second β-aminopropionic acid derivative is removed by distillation.

5. The process according to any of claims 1 or 4, wherein the acrylic acid derivative is acrylonitrile or an acrylic ester.

6. The process according to claim 4 or 5, wherein the secondary amine used in step (ii) is a low-boiling amine with a boiling point up to 100°C and step (iii) is carried out.

7. The process according to any of claims 1 to 6, wherein the secondary amine used in step (ii) is a di(C₁-C₄-alkyl)amine.

8. The process according to any of claims 1 to 7, wherein the secondary amine used in step (ii) is dimethylamine.

9. The process according to any of claims 1 to 8, wherein the amine of value is selected from the group consisting of 3,3'-dimethyl-4,4'-diaminodicyclohexylmethane (CAS No. 6864-37-5), 4,4'-diaminodicyclohexylmethane (CAS No. 1761-71-3), 4,4'-diaminodiphenylmethane (CAS No. 101-77-9), hexamethylenediamine (CAS No. 124-09-4), 2-methyl-pentamethylenediamine (CAS No. 15520-10-2), polyetheramine D 230 (CAS No. 9046-10-0) and 1,13-diamino-4,7,10-trioxatridecane (CAS No. 4246-51-9).

10. The process according to any of claims 1 to 9, wherein step (i) is carried out in the presence of water as a catalyst at a temperature of from 20 to 80°C.

## Revendications

1. Procédé de fabrication de dérivés de l'acide β-aminopropionique par réaction d'une amine primaire ou secondaire avec un dérivé d'acide acrylique,
**caractérisé en ce que**
(i) une première amine primaire ou secondaire est utilisée en tant qu'amine de valeur et est mise en réaction avec le dérivé d'acide acrylique, un mélange réactionnel qui contient un premier dérivé d'acide β-aminopropionique en tant que produit de valeur et également du dérivé d'acide acrylique non réagi étant obtenu,
(ii) le dérivé d'acide acrylique non réagi contenu dans le mélange réactionnel est mis en réaction avec une seconde amine secondaire en tant qu'amine de piégeage pour former pratiquement totalement un second dérivé d'acide β-aminopropionique, un mélange réactionnel qui contient le premier dérivé d'acide β-aminopropionique en tant que produit de valeur, le second dérivé d'acide β-aminopropionique et l'amine secondaire non réagie étant obtenu.

2. Procédé selon la revendication 1, **caractérisé en ce que** le dérivé d'acide acrylique est utilisé en excès stoechiométrique par rapport aux groupes amino primaires ou secondaires de l'amine de valeur.

3. Procédé selon la revendication 1, **caractérisé en ce que** le dérivé d'acide acrylique est utilisé en quantité sensiblement équimolaire ou en déficit stoechiométrique par rapport aux groupes amino primaires ou secondaires de l'amine de valeur.

4. Procédé selon l'une quelconque des revendications 1 à 3, **caractérisé en ce qu'**à partir du mélange réactionnel obtenu à l'étape (ii),
(iii) l'amine secondaire non réagie est éliminée par distillation
et/ou
(iv) le second dérivé d'acide β-aminopropionique est éliminé par distillation.

5. Procédé selon l'une quelconque des revendications 1 à 4, Caractérisé en ce le dérivé d'acide acrylique est l'acrylonitrile ou un ester de l'acide acrylique.

6. Procédé selon la revendication 4 ou 5, **caractérisé en ce que** l'amine secondaire utilisée à l'étape (ii) est une amine de faible point d'ébullition qui présente un point d'ébullition inférieur ou égal à 100 °C et l'étape (iii) est réalisée.

7. Procédé selon l'une quelconque des revendications 1 à 6, **caractérisé en ce que** l'amine secondaire utilisée à l'étape (ii) est une di-(C₁-C₄-alkyle)aminé.

8. Procédé selon l'une quelconque des revendications 1 à 7, **caractérisé en ce que** l'amine secondaire utilisée à l'étape (ii) est la diméthylamine.

9. Procédé selon l'une quelconque des revendications 1 à 8, **caractérisé en ce que** l'amine de valeur est choisie dans le groupe constitué du 3,3'-diméthyl-4,4'-diaminodicyclohexylmérhane (n° CAS 6864-37-5), le 4,4'-diaminodicyclohexylméthane (n° CAS 1761-71-3), le 4,4'-diaminodiphénylméthane (n° CAS 101-77-9), l'hexaméthylène diamine (n° CAS 124-09-4), la 2-méthylpentaméthylène diamine (n° CAS 15520-10-2), la polyéther amine D 230 (n° CAS 9046-10-0) et le 1,13-diamino-4,7,10-trioxatridécane (n° CAS 4246-51-9).

10. Procédé selon l'une quelconque des revendications 1 à 9, **caractérisé en ce que** l'étape (i) est réalisée en présence d'eau en tant que catalyseur à une température de 20 à 80 °C.
